# EUROPEAN PATENT SPECIFICATION

(11) **EP 3 541 942 B1**
(45) Date of publication and mention of the grant of the patent: **15.07.2020**
(21) Application number: 17838192.7
(22) Date of filing: 16.11.2017
(51) Int. Cl.: C12N 15/70, C12N 15/87

(54) **CONJUGATIVE VECTORS SELECTABLE BY FRUCTOOLIGOSACCHARIDES**
DURCH FRUCTOOLIGOSACCHARIDE AUSWÄHLBARE KONJUGATIVE VEKTOREN
VECTEURS DE CONJUGAISON POUVANT ÊTRE SÉLECTIONNÉS PAR DES FRUCTOOLIGOSACCHARIDES

(30) Priority: 17.11.2016 IT 201600116012
(43) Date of publication of application: 25.09.2019
(73) Proprietor: Istituto Superiore Di Sanita', 00161 Roma (RM) (IT); Universität Bern, 3012 Bern (CH)
(72) Inventor: CARATTOLI, Alessandra, 00161 Roma RM (IT); ENDIMIANI, Andrea, 3012 Bern (CH)
(74) Representative: Gitto, Serena
(86) International application number: PCT/IT2017/000256
(87) International publication number: WO 2018/092171

(56) References cited:
- WO-A2-02/18605
- US-A1- 2008 070 231
- MONIKA DOLEJSKA ET AL: "Complete sequences of IncHI1 plasmids carrying blaCTX-M-1 and qnrS1 in equine Escherichia coli provide new insights into plasmid evolution", JOURNAL OF ANTIMICROBIAL CHEMOTHERAPY., vol. 69, no. 9, 26 May 2014 (2014-05-26), pages 2388-2393, XP055374187, GB ISSN: 0305-7453, DOI: 10.1093/jac/dku172 cited in the application
- ALESSANDRA CARATTOLI ET AL: "Differentiation of IncL and IncM Plasmids Associated with the Spread of Clinically Relevant Antimicrobial Resistance", PLOS ONE, vol. 10, no. 5, 1 May 2015 (2015-05-01), page e0123063, XP055463986, DOI: 10.1371/journal.pone.0123063
- Neville Firth ET AL: "SECTION C @BULLET Gene Transfer: ConjugationStructure and Function of the F Factor and Mechanism of Conjugation" In: "Escherichia coli and Salmonella, Neidhard et al.", 1 January 1996 (1996-01-01), Washington, XP055373519, the whole document

## Description

The present invention relates to conjugative vectors selectable by fructooligosaccharides. Particularly, the present invention describes new DNA plasmid vectors capable of self-conjugation. These vectors do not contain antibiotic resistance as selection markers, but bacteria hosting them can be selected via their ability to grow on a special carbon source.

### Background of the invention

Plasmid vectors are circular molecules of DNA that can replicate autonomously in bacterial cells. They are widely used for genetic engineering, for instance to clone and express proteins of interest (e.g. vaccine antigens). The current genetic engineering technology is based on small plasmids (about 3-5 kb) that contain an origin of replication and a resistance marker and can be introduced into bacterial recipient strain by transformation. The transformed bacteria carrying the recombinant plasmid can propagate and express the genes cloned in the vector, but are not able to transfer the plasmid vector to other bacteria if the plasmid vector is not mobilized by a helper plasmid co-resident in the donor bacterial cell. The most commonly used marker for the selection of the bacteria that acquired the plasmid vector by transformation is a gene conferring resistance to an antibacterial drug. Therefore, bacteria that contain the vector can be selected due to the capacity to grow on agar plates containing the specific antibacterial drug. However, for some research and biotech applications, vectors without antibacterial resistance markers are needed. This applies to the study of antibacterial resistance mechanisms, or to the *in vivo* use of vectors to deliver proteins of interest such as vaccine antigens to the microbiota.

### Description of the invention

According to the present invention, a conjugative plasmid vector is provided. This comprises a set of genes operable within a first bacterium (donor) that enables transfer of said plasmid vector from said first bacterium to a second bacterium (recipient) via conjugation when said set of genes is present and functional in said first bacterium. The plasmid vector further comprises a selection marker operable in said first bacterium as well as in the second bacterium, wherein said selection marker enables the use of short-chain fructooligosaccharides (scFOS) as source of carbon in said first and second bacteria when the genes constituting said selection marker are transcribed in said first and second bacteria.

In the context of the present specification, the term "conjugation" is defined as a mechanism of horizontal gene transfer between bacterial cells involving cell-to-cell contact. During conjugation, the donor cell transfers a genetic element, most often a plasmid, to the recipient cell.

In the context of the present specification, the term "self-conjugative" or simply "conjugative" is defined as the ability of a plasmid vector to promote the transfer of a copy of itself from a donor bacterium to a recipient bacterium by conjugation.

In the context of the present specification, the term "scFOS" is defined as short-chain fructooligosaccharides, a group of linear fructose oligomers (n=1-5) that escape digestion in the human upper intestine and reach the colon and are not metabolized by most of the species belonging to the Enterobacteriaceae family (Bailey, et al., 1991. Poultry Science 70, 2433-2438; Spiegel et al., 1994 Food Technology 48, 85-89; Wang and Gibson, 1993, J. Applied Bacteriol. 75, 373-389; Hartemink et al., 1997 J Applied Microbiol 83 367-374). Examples for scFOS species are: 1-kestose, nystose, 1F-fructofuranosyl nystose.

According to the present invention, the selection marker is the *fos* operon, wherein the *fos* repressor is removed.

In the context of the present specification, the term "*fos* operon" is defined as a cluster of seven genes involved in the scFOS utilization pathway: *fosK, fosY, fosGH2, fosX, fosGH1, fosT,* and *fosR.*

*FosR* encodes the *fos* repressor, a specific transcriptional regulator of the LacI family, which represses expression of the other genes of the operon. In particular, *fosR* is defined as gene coding for FosR of plasmid pEQ1 (Escherichia coli strain T23, NCBI Ref Seq NC_023289.2) or homologues thereof.

According to the present invention, as mentioned above, the repressor element *fosR* has been removed from the *fos* operon. According to the present invention, it was demonstrated that the removal of *fosR* did not impair the expression of the *fos* operon but instead leads to increased expression of the other genes belonging to the *fos* operon and thus to an increased ability of a cell to use scFOS as carbon source. This modification improves the suitability of the *fos* operon as a selection marker, thus improving the ability to colonize the gut.

In the context of the present specification, *fosK* is defined as gene coding for FosK of plasmid pEQ1 (Escherichia coli strain T23, NCBI Ref Seq NC_023289.2) or homologues thereof.

In the context of the present specification, *fosY* is defined as gene coding for FosY of plasmid pEQ1 (Escherichia coli strain T23, NCBI Ref Seq NC_023289.2) or homologues thereof.

In the context of the present specification, *fosGH2* is defined as gene coding for FosGH2 of plasmid pEQ1 (Escherichia coli strain T23, NCBI Ref Seq NC_023289.2) or homologues thereof.

In the context of the present specification, *fosX* is defined as gene coding for FosX of plasmid pEQ1 (Escherichia coli strain T23, NCBI Ref Seq NC_023289.2) or homologues thereof.

In the context of the present specification, *fosGH1* is defined as gene coding for FosGH1 of plasmid pEQ1 (Escherichia coli strain T23, NCBI Ref Seq NC_023289.2) or homologues thereof.

In the context of the present specification, *fosT* is defined as gene coding for FosT of plasmid pEQ1 (Escherichia coli strain T23, NCBI Ref Seq NC_023289.2) or homologues thereof.

According to the present invention it has been surprisingly found that the laboratory strains of *Escherichia coli* carrying the plasmid vector of the present invention are able to colonize and prevail in a complex bacterial population such as those present in human feces in comparison to the same laboratory strain of *E*. *coli* which do not carry the plasmid vector. This occurs not only in a minimal medium containing scFOS, such as that used *in vitro,* but also in a complex environment such as that of the *in vitro* simulated gut system (Smet et al.,2010 J. Applied Microbiol110 541-549), obtained by fermentation in continuous culture of human feces in rich medium supplemented by scFOS.

Therefore, the plasmid vector of the present invention can be advantageously used in order to select the bacteria carrying the plasmid vector *in vitro* by using a minimal medium containing scFOS as the unique source of carbon, without any need of an antibacterial resistance marker. In addition, the plasmid vector of the present invention is able to promote the colonization of the bacteria carrying the plasmid vector *in vivo* when scFOS is provided, for instance with the selective diet. In this way, any genetic determinant inserted in the plasmid vector can be effectively expressed and can exert its function. In contrast, when scFOS is not provided, the bacteria carrying the plasmid vector of the present invention does not colonize the gut and is rapidly cured (i.e., eliminated from the intestinal tract).

In certain embodiments, mobile genetic elements (i.e., IS*903*) have been removed from the *fos* operon. The IS*903* mobile genetic elements flanking the *fos* operon have been described in (Dolejska et al., J Antimicrob Chemother 2014; 69: 2388-2393). Mobile genetic elements can integrate into the host bacterial genome, thereby causing the uncontrolled dissemination of the *fos* operon by transposition from the vector into the genome of unrelated recipient bacteria. Removal of mobile genetic elements from the *fos* operon is thus important to eliminate potential harmful effects of the plasmid vector.

In certain embodiments of the present invention, the first (donor) and the second (recipient) bacteria are selected from the Gram-negative species belonging to the Enterobacteriaceae family. The plasmid vector enables conjugation between different species of bacteria, specifically among Enterobacteriaceae and *Pseudomonas* spp.

According to the present invention, the genes enabling plasmid transfer via conjugation enable said transfer at temperatures relevant for laboratory and *in vivo* applications, specifically at temperatures higher than 25 °C, such as 30 - 37 °C. This temperature range ensures that conjugation can take place between bacteria resident to the human body, e.g. the gut, if these bacteria harbor said plasmid vector.

According to the present invention, the set of genes enabling plasmid transfer via conjugation comprises a gene selected from *excA* (encoding exclusion protein of the IncM type ExcA), *traY* (encoding typelV secretion protein of the IncM type TraY), *traX* (encoding relaxase TraX), *traW, traU, traR, traQ, traP, traO, traN, traM, traL, traK, trad* (encoding plasmid transfer ATPase TraJ), *traI, traH, pri* (encoding DNA primase Pri), *mobA* (encoding relaxase/mobilization DNase MobA), *mobB, tir* (encoding transfer inhibition protein Tir), *trbA, trbB* and *trbC.*

In the context of the present specification, *excA* is defined as gene coding for ExcA of plasmid R69 (Salmonella enterica subsp. enterica serovar Paratyphi B strain R69, GenBank KM406488), or homologues thereof.

In the context of the present specification, *traY* is defined as gene coding for TraY of plasmid R69 (Salmonella enterica subsp. enterica serovar Paratyphi B strain R69, GenBank KM406488), or homologues thereof.

In the context of the present specification, *traX is* defined as gene coding for TraX of plasmid R69 (Salmonella enterica subsp. enterica serovar Paratyphi B strain R69, GenBank KM406488), or homologues thereof.

In the context of the present specification, *traW* is defined as gene coding for TraW of plasmid R69 (Salmonella enterica subsp. enterica serovar Paratyphi B strain R69, GenBank KM406488), or homologues thereof.

In the context of the present specification, *traU* is defined as gene coding for TraU of plasmid R69 (Salmonella enterica subsp. enterica serovar Paratyphi B strain R69, GenBank KM406488), or homologues thereof.

In the context of the present specification, *traR* is defined as gene coding for TraR of plasmid R69 (Salmonella enterica subsp. enterica serovar Paratyphi B strain R69, GenBank KM406488), or homologues thereof.

In the context of the present specification, *traQ* is defined as gene coding for TraQ of plasmid R69 (Salmonella enterica subsp. enterica serovar Paratyphi B strain R69, GenBank KM406488), or homologues thereof.

In the context of the present specification, *traP* is defined as gene coding for TraP of plasmid R69 (Salmonella enterica subsp. enterica serovar Paratyphi B strain R69, GenBank KM406488), or homologues thereof.

In the context of the present specification, *traO* is defined as gene coding for TraQ of plasmid R69 (Salmonella enterica subsp. enterica serovar Paratyphi B strain R69, GenBank KM406488), or homologues thereof.

In the context of the present specification, *traN* is defined as gene coding for TraN of plasmid R69 (Salmonella enterica subsp. enterica serovar Paratyphi B strain R69, GenBank KM406488), or homologues thereof.

In the context of the present specification, *traM* is defined as gene coding for TraM of plasmid R69 (Salmonella enterica subsp. enterica serovar Paratyphi B strain R69, GenBank KM406488), or homologues thereof.

In the context of the present specification, *traL* is defined as gene coding for TraL of plasmid R69 (Salmonella enterica subsp. enterica serovar Paratyphi B strain R69, GenBank KM406488), or homologues thereof.

In the context of the present specification, *traK* is defined as gene coding for TraK of plasmid R69 (Salmonella enterica subsp. enterica serovar Paratyphi B strain R69, GenBank KM406488), or homologues thereof.

In the context of the present specification, *traJ* is defined as gene coding for TraJ of plasmid R69 (Salmonella enterica subsp. enterica serovar Paratyphi B strain R69, GenBank KM406488), or homologues thereof.

In the context of the present specification, *traI* is defined as gene coding for TraI of plasmid R69 (Salmonella enterica subsp. enterica serovar Paratyphi B strain R69, GenBank KM406488), or homologues thereof.

In the context of the present specification, *traH* is defined as gene coding for TraH of plasmid R69 (Salmonella enterica subsp. enterica serovar Paratyphi B strain R69, GenBank KM406488), or homologues thereof.

In the context of the present specification, *pri* is defined as gene coding for Pri of plasmid R69 (Salmonella enterica subsp. enterica serovar Paratyphi B strain R69, GenBank KM406488), or homologues thereof.

In the context of the present specification, *mobA* is defined as gene coding for MobA of plasmid R69 (Salmonella enterica subsp. enterica serovar Paratyphi B strain R69, GenBank KM406488), or homologues thereof.

In the context of the present specification, *mobB* is defined as gene coding for MobB of plasmid R69 (Salmonella enterica subsp. enterica serovar Paratyphi B strain R69, GenBank KM406488), or homologues thereof.

In the context of the present specification, *tir* is defined as gene coding for Tir of plasmid R69 (Salmonella enterica subsp. enterica serovar Paratyphi B strain R69, GenBank KM406488), or homologues thereof.

In the context of the present specification, *trbA* is defined as gene coding for TrbA of plasmid R69 (Salmonella enterica subsp. enterica serovar Paratyphi B strain R69, GenBank KM406488), or homologues thereof.

In the context of the present specification, *trbB* is defined as gene coding for TrbB of plasmid R69 (Salmonella enterica subsp. enterica serovar Paratyphi B strain R69, GenBank KM406488), or homologues thereof.

In the context of the present specification, *trbC* is defined as gene coding for TrbC of plasmid R69 (Salmonella enterica subsp. enterica serovar Paratyphi B strain R69, GenBank KM406488), or homologues thereof. The *traX, traY and excA* genes are part of the entry exclusion system of conjugative plasmids, which ensures that a bacterial cell does not harbor two closely related conjugative plasmids.

The genes *traW, traU, traR, traQ, traP, traO, traN, traM, traL, primase, traK, traJ, traI,* and *traH* of the *tra* transfer locus and the genes of the *trbA, trbB* and *trbC* of the *trb* transfer locus encode the conjugative transfer proteins.

According to the present invention, the set of genes enabling plasmid transfer via conjugation comprises genes of the *tra* and *trb* transfer loci.

According to the present invention, the set of genes enabling plasmid transfer via conjugation comprises all the genes *excA, traY, traX, traW, traU, traR, traQ, traP, traO, traN, traM, traL, traK, traJ, traI, traH, pri, mobA, mobB, tir, trbA, trbB* and *trbC.*

Specifically, the genes enabling plasmid transfer via conjugation are encoded by base pairs 12894-35539 and 59785-65769 of plasmid R69 (GenBank ID: KM406488). These base pairs comprise the genes *excA, traY, traX, traW, traiU, traR, traQ, traP, traO, traN, traM, traL, traK, traJ, traI, traH, pri, mobA, mobB, tir, trbA, trbB* and *trbC.*

R69 is a self-conjugative plasmid belonging to the IncM family. R69 has been selected as recipient vector of the *fos operon* because it has broad host range, including the capacity to replicate not only in Enterobacteriaceae, but also in bacteria of other genera such as for instance *Pseudomonas aeruginosa,* and conjugates at 37°C. The original pEQ1 plasmid carrying the *fos operon* belongs to the HI1 family, has a narrow host range limited to Enterobacteriaceae, conjugates only at 20-25°C, being the conjugation temperature sensitive (Taylor and Levine 1980 J Gen Microbiol 116: 475-484) and contains several resistance and virulence genes that cannot be removed without destroying the plasmid properties (Phan and Wain. 2008.J Infect Dev Ctries. 30;2(4):272-8). The original pEQ1 plasmid can be harmful for human and animal health if used as recipient vector.

According to the present invention, the plasmid vector, such as that based on R69 scaffold, as mentioned above, comprises *Fos operon* without *FosR*, in addition, it comprises R69 scaffold without any gene conferring resistance to any antibacterial drug. Presence of a gene conferring resistance to an antibacterial drug can be useful for selection purposes during the construction process (cloning) of a plasmid vector. However, for *in vivo* applications of a plasmid vector in animals or humans, presence of a gene conferring resistance to antibacterial drug is very unfavorable, since it poses the possible threat of the emergence of bacteria resistant to said antibacterial drug.

As mentioned above, the plasmid vector according to the present invention comprises a selection marker which enables the use of short-chain fructooligosaccharides (scFOS). Therefore, a gene conferring resistance to an antibacterial drug is not necessary as selection marker. This does not mean that the plasmid vector of the invention cannot comprise a gene conferring resistance to an antibacterial drug for other purposes which are different from selecting the bacteria carrying the plasmid vector.

For instance when an antibacterial therapy is necessary, a gene conferring resistance to the antibacterial drug used in the therapy can be inserted in the plasmid vector in order not to inhibit the colonization of the bacteria carrying the plasmid vector with a specific function, such as that exerted by a genetic determinant inserted in the plasmid vector.

In certain embodiment, the plasmid vector of the present invention is a large plasmid (>64 kb). The reason of this large size is that the plasmid vector can comprise a gene promoting replication and/or a gene promoting stability. The plasmid vector can comprise a large array of genes that are necessary for promoting broad host range replication (replicon encoding the replication initiating protein RepA, replication regulatory protein RepC), such as that encoded by base pairs 11238-12781 of plasmid R69 (GenBank ID: KM406488), stabilization in the bacterial cell (single-stranded DNA-binding protein SsB, antirestriction protein KIcA, transcriptional repressor protein KorC, DNA repair protein RadC, endonuclease Nuc, plasmid partitioning protein ParB, plasmid segregation protein ParA, resolvase ResD, restriction endonuclease Mrr_cat, antitoxin domain-containing protein ReIB, MucB, MucA, stable inheritance protein PemK (toxin), stable inheritance protein Peml (antitoxin), such as those encoded by base pairs 37715-59730 of plasmid R69 (GenBank ID: KM406488) and the above mentioned transfer from a bacterial donor to recipient cells (conjugation). Specific examples of plasmid vectors according to the first aspect of the invention are the plasmid vector pFOS1 and its derivatives that have been generated by the inventors as described in the examples. Once transformed in an *E. coli* strain, these plasmid vectors do not need further transformation to be transferred from one bacterial strain to another. This can be done by mating, i.e. co-cultivating donor and recipient strains into selective conditions. There is no need of helper plasmids, since the plasmid vectors are self-conjugative. The plasmid vectors can be used *in vivo* to bring genetic information within a complex bacterial population (i.e., the intestinal microbiota). They are adapted to the Enterobacteriaceae family and can change the bacterial host species by conjugation, for instance by reaching resident (autochthonous) *E. coli* colonizing the intestine of humans and animals, but also moving into other bacterial species since their host range is broad. These plasmid vectors can move from the bacteria that are administered (probiotic bacterial culture), into bacteria that are resident colonizers of the host gut.

New plasmid vectors for biotechnological use are provided by purified plasmid DNA of the pFOS-vector and a non-pathogenic bacterial suspension (*Escherichia coli* K12 DH5-alpha strain or *Escherichia coli* K12 JM83) carrying the pFOS-vector. This bacterial suspension can be grown in Luria-Bertani (LB) broth or plates in standard condition of bacterial cultivation or on M9 minimal medium containing scFOS as the unique carbon source for the selection of pFOS-positive transformants.

The pFOS-vector does not require resistance genes or the use of antimicrobial drugs to be positively selected.

The pFOS-vector contains a unique Hindlll site. This site can be used as restriction cloning site to insert every kind of gene expressing a function such as genetic determinants that need to be vehiculated and expressed by the pFOS-plasmid described in this invention.

The genetic determinant of interest should be amplified by PCR with primers carrying the Hindlll clamps to be ligated in the linearized HindIII-pFOS plasmid. The ligation mixture can be re-introduced into any kind of Escherichia coli cell by electro-transformation or in chemically competent cells. The transformants can be selected on M9 minimal medium containing scFOS as the unique carbon source. The positive transformants carrying the engineered pFOS vector can be then used to test the properties of the cloned insert. The most innovative use of this vector is the possibility to test the properties of the cloned insert directly in *in vivo* animal models. A suspension of the transformants carrying the engineered plasmid of this invention can be administered to an animal model sustaining the colonization in the gut by administering scFOS sugars in the diet of the animals (such as the Horse digest-plus product containing scFOS produces by Baileys: https://www.baileyshorsefeeds.co.uk/products/digest-plus).

According to a further aspect of the invention, a cell comprising the plasmid vector according to the invention is provided.

In certain embodiments, the cell is a bacterial cell.

In certain embodiments, the bacterial cell can colonize the gut of animals or humans.

The bacterial cell can be selected by a diet enriched with scFOS.

The scFOS are established food supplements marketed for restoration of the intestinal flora in patients (including children) and animals and are accepted as prebiotic by FDA in the USA. The presence of scFOS provides a selective advantage for bacterial cells carrying the plasmid vector containing the *fos* operon, since these bacteria are able to metabolize scFOS, while the other Enterobacteriaceae of the microbiota cannot. Therefore, according to the experimental results herewith presented, it is expected that in the intestinal ecosystem the enterobacterial proportion will change and the bacteria expressing the *fos* operon will transiently prevail and colonize the host intestine. The same results can be obtained also by topical use of the bacteria carrying the plasmid vector of the invention on the mucosa.

For biological and ecological safety reasons, pFOS plasmids are intended to be inherently unstable to be rapidly cleared from their host bacteria and the environment when the scFOS supplemented diet is suspended. The pFOS conjugative vectors are expected to horizontally transfer between intestinally co-colonizing bacteria by conjugative exchange, reaching the endogenous bacteria.

According to the invention, a combination is provided, comprising
a. a non-pathogenic suspension for example an *Escherichia coli* DH5-α or JM83 laboratory bacterial cell containing the pFOS vector; and
b. a pharmaceutical or dietary formulation of scFOS.

The bacteria carrying the plasmid vector, according to the experimental results herewith presented, are expected to colonize the intestine of humans and animals *in vivo,* taking selective advantage of the diet enriched by scFOS. scFOS will thus serve a prebiotic. The prebiotic oligosaccharide scFOS can augment colonization of beneficial intestinal species that encode the *fos* operon. In the last stage, the pFOS vector transferred from donor to target strain by conjugation and brings information to the indigenous colonizing bacteria (Mimee et al., 2016. Adv Drug Deliv Rev.;105:44-54).

There will be a huge field of application for such a safe vector that can be introduced by conjugation also in bacteria that carry resistance to multiple antibiotic classes and that are often inaccessible to transformation or conjugation experiments because of the lack of a suitable resistance marker for the selection. The scFOS selection is therefore by itself an innovative and promising factor for molecular engineering of bacteria.

The delivery of vaccine antigens to the microbiota can be obtained by cloning the gene encoding the antigen within the vector. The antigens are expected to be expressed and delivered in the microbiota by the conjugative vector in the population resident in the mucosa or in the gut. The vector encoding the antigen can colonize the mucosa or the gut, favoring the host immunological response towards the cloned antigen (Lex and Azizi, 2017 Expert Rev Vaccines. Oct 25:1-3).

New therapeutic strategies can be applied based on the use of the CRISPR systems. The Type II CRISPR system has been already used for designing new antimicrobial strategies. Phages have been used to deliver a CAS9 system designed to target specific bacterial DNA sequences(Citorik et al., 2014 Nature Biotechnology 32:1141-5). However, phages were an inefficient delivery system. The Cas9, trans-activating crRNA, and specific arrays can be cloned within the pFOS vector, exploring the efficiency of antibacterial delivery to the microbiota. The antibacterial CRISPRs are expected to be expressed and delivered in the microbiota by the conjugative vector in the population resident in the mucosa or in the gut, targeting specific bacterial pathogens within the gut on basis of designed CRISPR arrays.

New strategies can be applied to cure resistance plasmids within bacterial pathogens, antitoxin genes and incompatibility determinants can be cloned within the vector (Kamruzzaman et al., 2017 PLoS One. 2017 12(2):e0172913). The antiplasmid functions are expected to be expressed and delivered in the microbiota by the conjugative vector in the population resident in the mucosa or in the gut, causing the destabilization and cure of resistance plasmids within bacteria colonizing the gut, restoring their antimicrobial susceptibility, without perturbing the bacterial ecosystem within the gut but counteracting the spread of resistance mediated by resistance plasmids.

### Description of figures and tables

**Figure 1** shows the *fos* operon from plasmid pEQ1, a cluster of genes involved in the scFOS utilization pathway. The position of the primers used for amplification of the *fos* operon and the position of the IS*903* mobile elements, eliminated during the cloning of the *fos* operon are indicated by small and large arrows, respectively
**Figure 2** shows the amplification of the *fos operon* from the pEQ1 natural plasmid to be cloned into the Hindlll site of the pUC19 vector.
**Figure 3** shows the cloning of the HIndIII *fos* operon fragment in the pUC19 vector, obtaining two clones named A21 and A21bis with the fragment cloned in opposite orientation with respect to the pUC19 multi-cloning site.
**Figure 4** shows the deletion of the *fosR* repressor from the *fos* operon by self-ligation on the Smal site of the pUC19 and the Smal site occurring within the *fosR* gene, generating the recombinant pUC19 derivative named A21 ΔfosR clone.

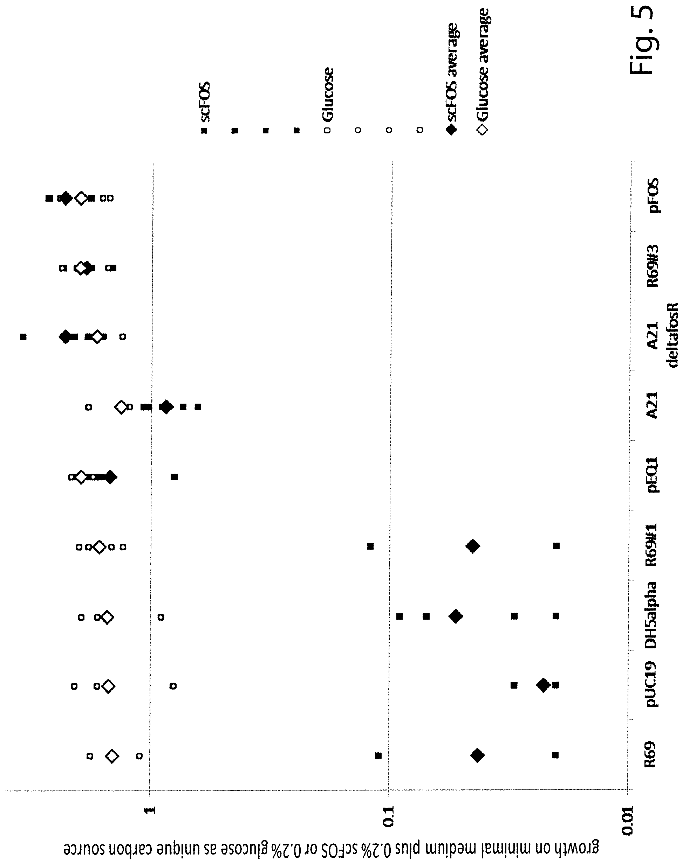

**Figure 5** shows a test of growth of E. coli transformants DH5a in M9 Minimal liquid medium containing 0.2% scFOS (FOS) or 0.2% glucose (GLU) as the unique carbon sources, respectively. pUC19 does not contain the *fos* operon, A21 is a derivative of pUC19 that contains the original *fos* operon (without mobile elements) and A21ΔfosR contains the modified *fos* operon (without mobile elements and the *fosR* repressor gene). R69 does not contain the *fos* operon, pEQ1 contains the original *fos* operon, pFOS1 contains the modified *fos* operon and R69#3 contains the modified *fos* operon and in addition a kanamycin resistance gene. Measurement of optical density in McFarland reached by cultures after 24 hrs of growth (repeated trice) and the average of the results obtained in the three experiments of growth using glucose or scFOS as carbon sources was calculated and plotted as *glucose average* value and *scFOS average* value, respectively.
**Figure 6** shows the modification of the vector R69 by elimination of the ampicillin and tetracycline resistance genes obtaining the intermediate R69#1 vector, which still carries the kanamycin resistance determinant.
**Figure 7** shows how the pFOS1 was constructed, cloning the Smal fragment containing the *fos* operon without the *fosR* gene in the scaffold of the R69#1 plasmid, obtaining the R69#3 vector. R69#3 was further modified by elimination of the kanamycin resistance element by recircularization of the Hindlll digested R69#3 plasmid, obtaining the pFOS1 vector.

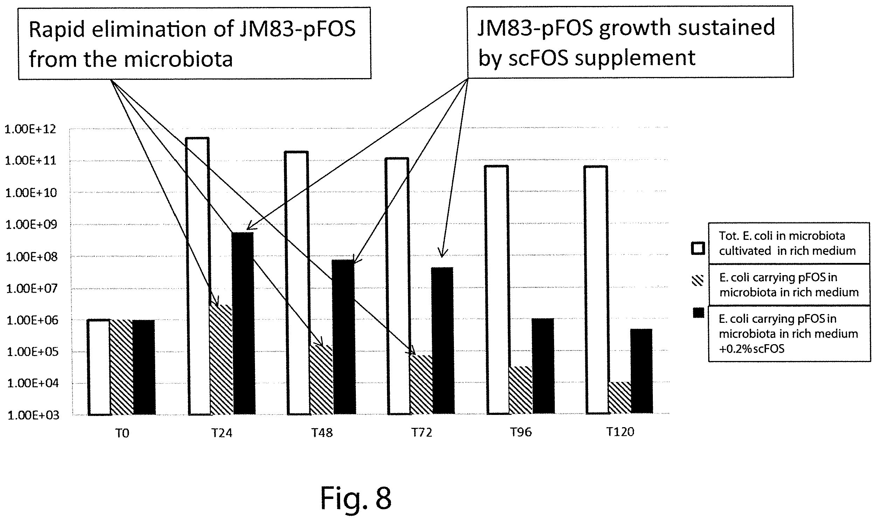

**Figure 8** shows the results of tests of growth into the human intestinal microbiota of *E*. *coli* carrying pR69#3 vector in continuous flow culture system. Co-cultivation results of human faeces and *E*. *coli* JM83 (lactose mutant) carrying the R69#3 vector, carrying the fos operon, in BHI medium with or without 0.2% scFOS in a 3-L glass fermentation vessel, operated under the control of a BioFlo unit (New Brunswick Scientific, NJ, USA). The *E. coli* JM83-R69#3 was monitored by taking aliquots of the continuous culture and plating dilution on MacConkey Agar with 30 mg/L kanamycin. Kanamycin resistance in R69#3 was used for simplifying the analysis in the fermentor, simplifying the measurement of CFU of R69#3-containing cells, but kanamycin has not been added to the cultures and not used as selection marker in these experiments. Kanamycin resistance has been removed from the final pFOS1 vector. These experiments were repeated three times with or without scFOS supplements.

### Examples

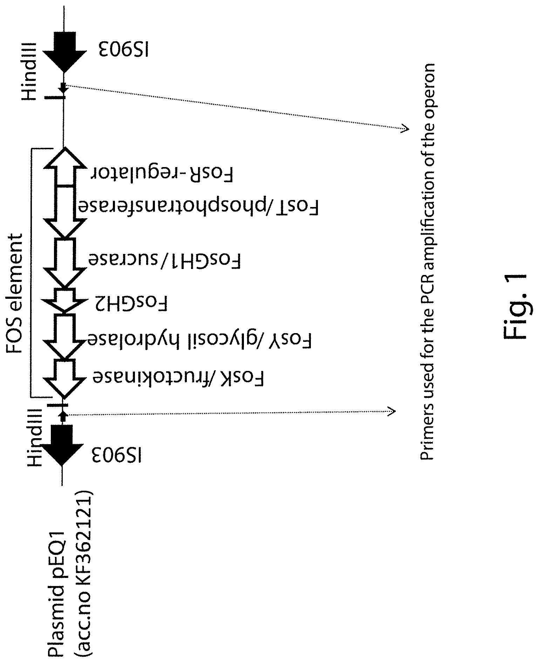

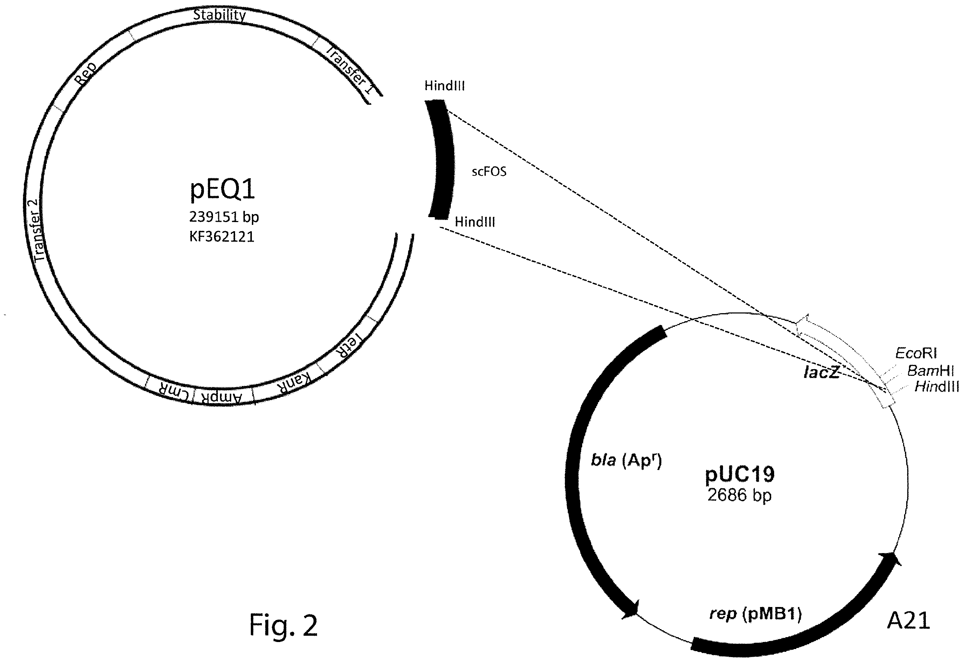

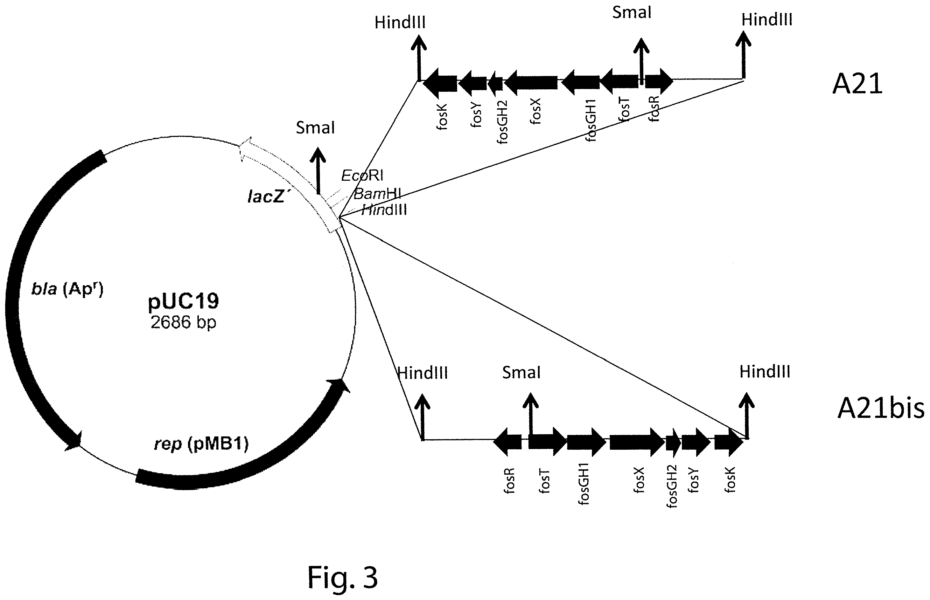

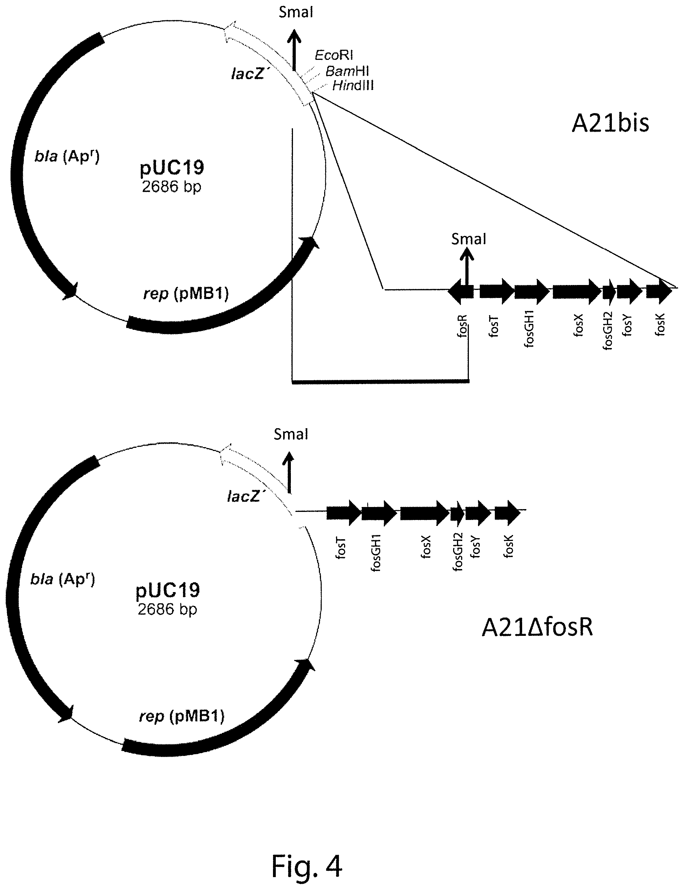

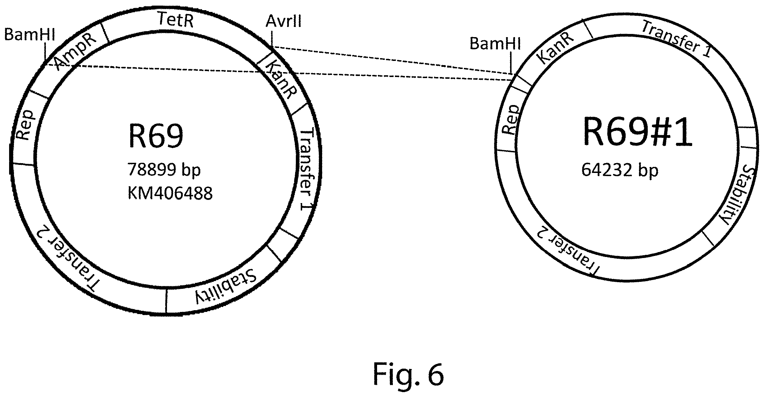

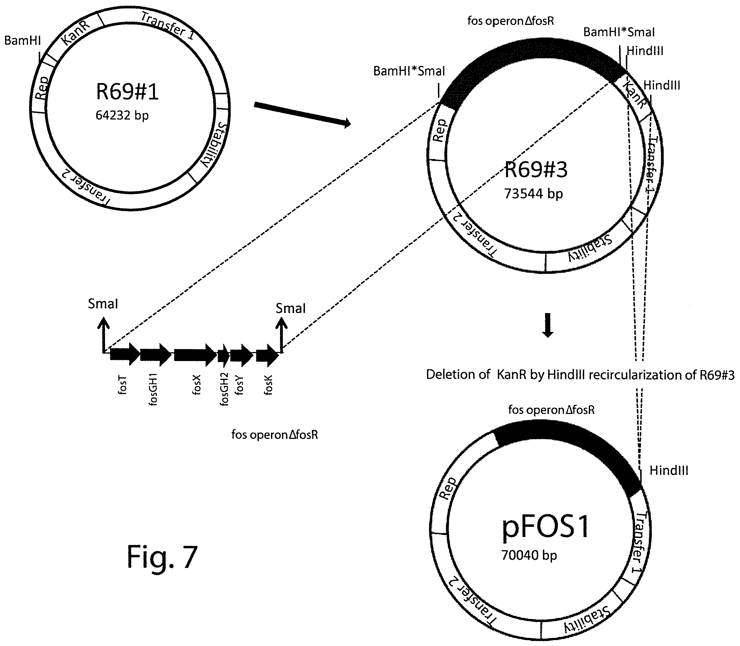

The invention is further illustrated by the following examples and figures, from which further embodiments and advantages can be drawn. These examples are meant to illustrate the invention, but not to limit its scope. To realize the invention, the researchers performed the following steps:
1- Transfer of the *fos* operon conferring ability to grow on scFOS from the natural plasmid pEQ1 (Dolejska et al., J Antimicrob Chemother 2014; 69: 2388-2393) to a cloning vector pUC19 (New England Biolabs Inc.). In this passage the operon was amplified by PCR without the mobile genetic elements (IS*903* elements) constituting potential uncontrolled transferability factors of the *fos* operon (Figure 1). The amplicon was digested with the restriction enzyme Hindlll and cloned in the unique Hindlll site of the multi-cloning site of the vector pUC19 (Figure 2), obtaining the intermediate clone named A21 and A21bis with the insert cloned in opposite orientations (Figure 3). The deletion of the *fosR* repressor from the *fos* operon by self-ligation of the A21bis on the Smal site, between the Smal site of the pUC19 vector and the naturally occurring Smal site present in the *fos operon* immediately before the *fosR* gene, generating the recombinant pUC19 derivative named A21ΔfosR, lacking the *fosR* gene(Figure 4).
   Molecular elimination of the *fosR* repressor gene from the *fos* operon, resulted in increased expression of the remaining genes of the *fos* operon and increased ability of bacteria containing the plasmid vector to grow on scFOS as the unique source of carbons. Tested initially on the two pUC19 recombinant plasmids A21 and A21ΔfosR (Figures 4, 5).
   Measurement of optical density in McFarland Units reached by cultures after 24 hrs of growth (repeated trice). The growth is measured in a DensiCHEK plus bioMérieux instrument after standardization with 0.0 and 2.0 McFarland references. Bacterial suspension turbidity is considered as a meaning of optimal growth (>1.0 McFarland) and no growth (<0.5 McFarland).
   In this experiment A21ΔfosR clone demonstrates an average of 2.6 times increased capacity of growth on scFOS as the unique source of carbons than A21, showing a better growth also with respect to the glucose used as the unique carbon source. The experiment demonstrated that the elimination of the *fosR* gene from the *fos* operon was useful for the production of a vector based on the scFOS positive selection. These experiments also demonstrated that the *fos* operon was able to be expressed also without the presence of the *fosR* gene.
   For this and other experiments the minimal medium M9 was prepared as described in: http://cshprotocols.cshlp.org/content/2010/8/pdb.rec12295.short.
   M9 medium was supplemented with 1 mg/L thiamine hydrochloride B1 (SIGMA Aldrich) for complementation of thiamine auxotrophy of the DH5-α recipient bacterial cells (Thermofisher Scientific) or with 250 mg/L proline (SIGMA Aldrich) for proline auxotrophy of JM83 recipient bacterial cells (ATCC; http://www.atcc.org), respectively.
   As carbon sources the following sugars were used:
   - 5 mg/ml Nystose [β-D-Fruf-(2->1)-β-D-Fruf-(2->1)-β-D-Fruf-(2<->1)-α-D-Glup] (SIGMA Aldrich)
   - 5 mg/L Kestose [β-D-Fruf-(2→1)-β-D-Fruf-(2→1)-α-D-Glup] (SIGMA Aldrich)
   - 0.2% scFOS crude extract marketed as prebiotic for animal nutrition (https://www.baileyshorsefeeds.co.uk/products/digest-plus)
   - 0.2% glucose (SIGMA Aldrich).
2- Elimination of resistance genes (conferring ampicillin/tetracycline resistance) from the self-conjugative plasmid R69, by restriction with BamHI and AvrII, repairing the protruding extremities with Klenow DNA polymerase and circularizing the DNA molecule by self-ligation in diluted conditions. Ligation mixture was used to transform chemically competent *Escherichia coli* DH5a cells. Transformants were initially screened on 30 mg/L kanamycin (Sigma-Aldrich) containing LB agar plates. By replica plating on 50 mg/L ampicillin (Sigma-Aldrich) containing LB agar plates, kanamycin resistant-ampicillin susceptible strains were identified. Plasmid preparation and restriction analysis confirmed the elimination of the ampicillin and tetracycline resistance region from the R69 plasmid. Tetracycline susceptibility was also verified in liquid culture after induction of *tetA* gene expression by incubation of 45 min at 37°C with 1 mg/L of tetracycline (Sigma-Aldrich) followed by overnight incubation in 25 mg/L tetracycline. The plasmid obtained was named R69#1 intermediate vector (Figure 6 scheme of the cloning).
3- Cloning of the modified *fos* operon ΔfosR into the self-conjugative plasmid R69#1 intermediate vector was obtained by ligation of the Smal restriction fragment purified from the A21 clone into the BamHI R69#1 linearized vector. Before ligation was done, the BamHI protruding extremities of R69#1 were made blunt ends by Klenow polymerase (Life Technologies) incubation. Ligation mixture was used to transform chemically competent DH5a cells and transformants were selected on LB agar plates containing 30 mg/L kanamycin. Plasmid DNA from transformants was purified and checked by restriction with the Pvull enzyme and by PCR amplification for the presence of the *fos* operon devising primers on the *fosT* gene*.* The vector obtained was named R69#3 vector carrying the *fos* operon and the kanamycin resistance (Figure 7).
4- The elimination of kanamycin resistance gene from the R69#3 vector carrying the *fos* operon was performed by restriction of R69#3 with HindIII and self-ligation in diluted conditions. Ligation mixture was used to transform DH5a competent cells. Transformants were selected on M9 minimal medium agar plates containing scFOS as the unique source of carbon. Colonies were obtained after 48 hrs of growth at 37°C. Plasmid DNA was extracted from transformants and checked for restriction. PCR of the *fosT* gene was used to test for the presence of the *fos operon.* PCR for the replicase gene *repA* of the R69 plasmid was used for confirming the presence of the R69-derivative scaffold. Transformants were tested for ampicillin, tetracycline, and kanamycin resistance; formally the resistance present in the original R69 plasmids and the construct was susceptible to all drugs. The vector was named pFOS1 (Figure 7).
5- pFOS1 was tested for conjugative properties by mating with Escherichia coli JM83 recipient cells. JM83 recipient and DH5a donor strains were discriminated on the basis of their different auxotrophic phenotypes thiamine deficiency for DH5a and proline deficiency for JM83: Conjugation from the DH5-alha to JM83 was obtained at the 1x10⁻³ frequency (1 positive transformant obtained on 10³ recipient cells tested)
6- Evaluation of the capacity of all constructs containing the *fos* operon (i.e., R69#3, pFOS, A21, A21deltafosR, the original pEQ1 natural plasmid, R69#1) to growth on minimal medium containing scFOS as the unique source of carbons, verifying that this property does not subsist in the bacterial cell without the vector (DH5alpha), in pUC19, R69, R69#1 intermediate recombinants (Figure 5) but is conferred specifically by the cloned *fos operon* with a performance that is higher than that observed for the plasmid pEQ1, the natural donor of the *fos operon,* when cloned in the R69#3 vector.
7- Analysis of faecal bacterial dynamics in presence or absence of scFOS (Figure 8). 1 gr of pooled fresh faeces from three healthy volunteers were inoculated in 1 L of BHI rich medium with or without 0.2% scFOS in a 3-L glass fermentation vessel, operated under the control of a BioFlo unit (New Brunswick Scientific, NJ, USA). At the faecal suspension, 10⁶ CFU E. coli JM83 (lactose mutant) carrying the R69#3 vector comprising the *fos* operon and the kanamycin resistance, were added and the culture was maintained for 96 hrs with moderate agitation under microaerophilic conditions. Immediately after inoculation of the E. coli JM83, a sample was taken to calculate the start number of donor strains in the fermentation system. The cultivable microbiota was monitored and counted by taking aliquots of the continuous culture and plating dilutions on MacConkey agar with and without 30 mg/L kanamycin. Lactose negative/kanamycin-resistant colonies were counted to measure the capacity of the E. coli JM83-R69#3 culture to be maintained within the cultivable microbiota. These experiments were repeated three times in presence of BHI only and three times in presence of BHI-plus scFOS. The experiments clearly demonstrated that the *E. coli* JM83 is highly stable in cultivation with BHI and scFOS, while it was diluted and lost in 72 hrs without the positive selection exerted by the scFOS. scFOS can be utilized as carbon source by the *E. coli* JM83 which carry vector R69#3 containing the *fos* operon and this can give to the *E*. *coli* a transitory selective advantage that allows the colonization of the cultivable microbiota.
8- Identification of natural transconjugants occurring in the fermentor, due to the spontaneous conjugation promoted by R69#3. Since the donor JM83 was lactose-negative, the transfer of R69#3 was identified detecting in the cultivated microbiota the presence of in lactose-positive *E. coli* strains belonging to the original faecal population after 48 hrs of incubation in the fermentor. The lac-positive strains were typed by PCR with *fosT* and *repA* of R69, and by purification of plasmid DNA and comparison with the original R69#3 plasmid. Natural *E*. *coli* strains were found to have received the R69#3 vector, carrying the *fos operon.* This is the demonstration that the R69#3 *fos operon* vector was transferred by conjugation, moving from the original laboratory JM83 strain into the complex bacterial population reaching a recipient cell of fecal origin. The results serve as proof of principle on the potential activity of pFOS-plasmids to be used as vectors for microbiota engineering. Basically, this kind of self-conjugative vectors can promote the colonizing of a complex microbial population bringing new genetic determinants into the resident bacterial flora.

### Conclusions:

- We constructed self-conjugative vector that does not confer antimicrobial resistance to the bacterial host, but it confers the capacity to utilize the scFOS as carbon source.
- We demonstrated in conditions simulating the colonization of faecal microbiota that non-pathogenic *E*. *coli* bacteria carrying the pFOS vectors can successfully colonize the intestinal microbiota when the medium is supplemented with scFOS and be transferred by conjugation into resident recipient bacteria, whose growth can be sustained by the scFOS supplement. This kind of vectors can then exponentially amplify the message driven by the vector and used to transiently but efficiently bring novel genetic information into the microbiota.

## Claims

1. A plasmid vector comprising
a. a set of genes operable within a first bacterium, wherein said set of genes enables transfer of said plasmid vector from said first bacterium to a second bacterium via conjugation and wherein said set of genes enabling plasmid transfer via conjugation comprises all the following genes: *excA* encoding exclusion protein of the IncM type, *traY* encoding typeIV secretion protein of the IncM type, *traX* encoding relaxase, *traW, traU, traR, traQ, traP, traO, traN, traM, traL, traK, trad* encoding plasmid transfer ATPase, *traI, traH, pri* encoding DNA primase, *mobA* encoding relaxase/mobilization DNase, *mobB, tir* encoding transfer inhibition protein, *trbA, trbB* and *trbC,* and
b. a selection marker operable in said first bacterium and in said second bacterium, wherein said selection marker enables the use of short-chain fructooligosaccharides as carbon source in said first bacterium and second bacterium and is *fos* operon encoding fructokinase FosK, FosY, glycoside hydrolase FosGH2, FosX, glycoside hydrolase FosGH1, sugar transporter FosT and not FosR.

2. The plasmid vector according to claim 1, wherein said first and said second bacterium are selected from the species belonging to the Enterobacteriaceae family.

3. The plasmid vector according to any one of the above claims, wherein said set of genes enabling plasmid transfer via conjugation is encoded by base pairs 12894-35539 and 59785-65769 of plasmid R69 (GenBank ID: KM406488).

4. The plasmid vector according to anyone of claims 1-3, wherein said plasmid vector comprises a set of genes enabling plasmid replication which comprises the following genes: RepA replication protein, RepC replication regulatory protein.

5. The plasmid vector according to any one of the above claims, wherein said set of genes enabling plasmid replication is encoded by base pairs 11238-12781 of plasmid R69 (GenBank ID: KM406488),

6. The plasmid vector according to anyone of claims, wherein said plasmid vector comprises a set of genes enabling plasmid stability which comprises one or more or all the following genes: single-stranded DNA-binding protein SsB, antirestriction protein KlcA, transcriptional repressor protein KorC, DNA repair protein RadC, endonuclease Nuc, plasmid partitioning protein ParB, plasmid segregation protein ParA, resolvase ResD, restriction endonuclease Mrr_cat, antitoxin domain-containing protein ReIB, MucB, MucA, stable inheritance protein PemK (toxin), stable inheritance protein Peml (antitoxin).

7. The plasmid vector according to any one of the above claims, wherein said set of genes enabling plasmid replication is encoded by base pairs 37715-59730 of plasmid R69 (GenBank ID: KM406488).

8. The plasmid vector according to any one of the above claims, wherein said plasmid vector does not comprise any gene conferring resistance to an antibacterial drug.

9. A cell comprising the plasmid vector according to any one of the above claims.

10. The cell according to claim 9, wherein said cell is a bacterial cell.

11. A process of using said plasmid vector according to any one of claims 1 to 10 for the cloning and expression of genes.

12. Pharmaceutical composition comprising the cell as defined in anyone of claims 9-10 in association with one or more pharmaceutically acceptable excipients and/or adjuvants.

13. Kit comprising a) the pharmaceutical composition as defined in claim 12 and b) a pharmaceutical or dietary composition comprising short-chain fructooligosaccharides.

## Patentansprüche

1. Plasmidvektor, umfassend
a) einen Satz Gene, der innerhalb eines ersten Bakteriums funktionsfähig ist, wobei der Satz Gene den Transfer des Plasmidvektors von dem ersten Bakterium zu einem zweiten Bakterium mittels Konjugation ermöglicht und wobei der Satz Gene, der den Plasmidtransfer mittels Konjugation ermöglicht, alle der folgenden Gene umfasst: *excA,* das Ausschlussprotein des IncM-Typs codiert, *traY,* das Typ-IV-Sekretionsprotein des IncM-Typs codiert, *traX,* das Relaxase codiert, *traW, traU, traR, traQ, traP, traG, traN, traM, traL, traK, traJ,* die Plasmidtransfer-ATPase codieren, *traI, traH, pri,* die DNA-Primase codieren, *mobA,* das Relaxase/Mobilisations-DNase codiert, *mobB, tir,* die Transferinhibitionsprotein codieren, *trbA, trbB* und *trbC,* und
b) einen Selektionsmarker, der in dem ersten Bakterium und dem zweiten Bakterium funktionsfähig ist, wobei der Selektionsmarker die Verwendung kurzkettiger Fructooligosaccharide als Kohlenstoffquelle in dem ersten Bakterium und dem zweiten Bakterium ermöglicht und ein *fos*-Operon ist, das Fructokinase FosK, FosY, Glycosidhydrolase FosGH2, FosX, Glycosidhydrolase FosGH1, Zuckertransporter FosT und Non-FosR codiert.

2. Plasmidvektor nach Anspruch 1, wobei das erste und zweite Bakterium aus der Spezies ausgewählt sind, die zur Familie der Enterobacteriaceae gehört.

3. Plasmidvektor nach einem der vorstehenden Ansprüche, wobei der Satz Gene, der den Plasmidtransfer mittels Konjugation ermöglicht, durch die Basenpaare 12894-35539 und 59785-65769 von Plasmid R69 (GenBank-ID: KM406488) codiert wird.

4. Plasmidvektor nach einem der Ansprüche 1-3, wobei der Plasmidvektor einen die Plasmidreplikation ermöglichenden Satz Gene umfasst, der die folgenden Gene umfasst: RepA-Replikationsprotein, RepC-Replikationsregulationsprotein.

5. Plasmidvektor nach einem der vorstehenden Ansprüche, wobei der die Plasmidreplikation ermöglichende Satz Gene durch die Basenpaare 11238-12781 von Plasmid R69 (GenBank-ID: KM406488) codiert wird.

6. Plasmidvektor nach einem der vorstehenden Ansprüche, wobei der Plasmidvektor einen die Plasmidstabilität ermöglichenden Satz Gene umfasst, der ein oder mehrere oder alle der folgenden Gene umfasst: Einzelstrang-DNA-bindendes Protein SsB, Antirestriktionsprotein KlcA, Transkriptionsrepressorprotein KorC, DNA-Reparaturprotein RadC, Endonuklease Nuc, Plasmidpartitionierungsprotein ParB, Plasmidsegregationsprotein ParA, Resolvase ResD, Restriktionsendonuklease Mrr_cat, Antitoxindomänen enthaltendes Protein RelB, MucB, MucA, stabiles Vererbungsprotein PemK (Toxin), stabiles Vererbungsprotein PemL (Antitoxin).

7. Plasmidvektor nach einem der vorstehenden Ansprüche, wobei der die Plasmidreplikation ermöglichende Satz Gene durch die Basenpaare 37715-59730 von Plasmid R69 (GenBank-ID: KM406488) codiert wird.

8. Plasmidvektor nach einem der vorstehenden Ansprüche, wobei der Plasmidvektor kein Gen umfasst, das Resistenz gegen ein antibakterielles Arzneimittel verleiht.

9. Zelle, umfassend den Plasmidvektor nach einem der vorstehenden Ansprüche.

10. Zelle nach Anspruch 9, wobei die Zelle eine Bakterienzelle ist.

11. Verfahren zur Verwendung des Plasmidvektors nach einem der Ansprüche 1 bis 10 zum Klonieren und Exprimieren von Genen.

12. Pharmazeutische Zusammensetzung, umfassend die Zelle wie in einem der Ansprüche 9-10 definiert in Verbindung mit einem oder mehreren pharmazeutisch akzeptablen Arzneimittelträgern und/oder Adjuvantien.

13. Kit, umfassend a) die pharmazeutische Zusammensetzung wie in Anspruch 12 definiert und b) eine pharmazeutische oder diätetische Zusammensetzung, die kurzkettige Fructooligosaccharide umfasst.

## Revendications

1. Vecteur plasmidique comprenant
a. un ensemble de gènes utilisables à l'intérieur d'une première bactérie, dans lequel ledit ensemble de gènes permet le transfert dudit vecteur plasmidique depuis ladite première bactérie vers une seconde bactérie par conjugaison et dans lequel ledit ensemble de gènes permettant le transfert d'un plasmide par conjugaison comprend tous les gènes suivants : *excA* codant pour une protéine d'exclusion du type IncM, *traY* codant pour une protéine de sécrétion de type IV du type IncM, *traX* codant pour une relaxase, *traW, traU, traR, traQ, traP, traG, traN, traM, traL, traK, traJ* codant pour une ATPase de transfert de plasmide, *traI, traH, pri* codant pour une ADN primase, *mobA* codant pour une relaxase/ADNase de mobilisation, *mobB, tir* codant pour une protéine d'inhibition de transfert, *trbA, trbB* et *trbC,* et
b. un marqueur de sélection utilisable dans ladite première bactérie et dans ladite seconde bactérie, dans lequel ledit marqueur de sélection permet l'utilisation de fructooligosaccharides à chaîne courte comme source de carbone dans lesdites première bactérie et seconde bactérie et est l'opéron *fos* codant pour la fructokinase FosK, FosY, la glycoside hydrolase FosGH2, FosX, la glycoside hydrolase FosGH1, le transporteur de sucre FosT et pas pour FosR.

2. Vecteur plasmidique selon la revendication 1, dans lequel ladite première et ladite seconde bactérie sont sélectionnées parmi les espèces appartenant à la famille des entérobactéries.

3. Vecteur plasmidique selon l'une quelconque des revendications précédentes, dans lequel ledit ensemble de gènes permettant un transfert de plasmide par conjugaison est codé par les paires de bases 12 894 à 35 539 et 59 785 à 65 769 du plasmide R69 (identifiant GenBank : KM406488).

4. Vecteur plasmidique selon l'une quelconque des revendications 1 à 3, dans lequel ledit vecteur plasmidique comprend un ensemble de gènes permettant une réplication plasmidique qui comprend les gènes suivants : protéine de réplication RepA, protéine régulatrice de réplication RepC.

5. Vecteur plasmidique selon l'une quelconque des revendications précédentes, dans lequel ledit ensemble de gènes permettant une réplication plasmidique est codé par les paires de bases 11 238 à 12 781 du plasmide R69 (identifiant GenBank : KM406488).

6. Vecteur plasmidique selon l'une quelconque des revendications, dans lequel ledit vecteur plasmidique comprend un ensemble de gènes permettant une stabilité plasmidique qui comprend un ou plusieurs ou la totalité des gènes suivants : protéine SsB de liaison à l'ADN simple brin, protéine KIcA d'antirestriction, protéine KorC de répression de la transcription, protéine RadC de réparation de l'ADN, endonucléase Nuc, protéine ParB de partage de plasmide, protéine ParA de ségrégation plasmidique, résolvase ResD, endonucléase de restriction Mrr_cat, protéine RelB contenant un domaine antitoxine, MucB, MucA, protéine PemK d'héritage stable (toxine), protéine Peml d'héritage stable (antitoxine).

7. Vecteur plasmidique selon l'une quelconque des revendications précédentes, dans lequel ledit ensemble de gènes permettant une réplication plasmidique est codé par les paires de bases 37 715 à 59 730 du plasmide R69 (identifiant GenBank : KM406488).

8. Vecteur plasmidique selon l'une quelconque des revendications précédentes, dans lequel ledit vecteur plasmidique ne comprend aucun gène conférant une résistance à un médicament antibactérien.

9. Cellule comprenant le vecteur plasmidique selon l'une quelconque des revendications précédentes.

10. Cellule selon la revendication 9, dans laquelle ladite cellule est une cellule bactérienne.

11. Procédé d'utilisation dudit vecteur plasmidique selon l'une quelconque des revendications 1 à 10 pour le clonage et l'expression de gènes.

12. Composition pharmaceutique comprenant la cellule telle que définie dans l'une quelconque des revendications 9 et 10 en association avec un ou plusieurs excipients et/ou adjuvants pharmaceutiquement acceptables.

13. Kit comprenant a) la composition pharmaceutique telle que définie dans la revendication 12 et b) une composition pharmaceutique ou alimentaire comprenant des fructooligosaccharides à chaîne courte.
